# FASCICULE DE BREVET EUROPEEN

(11) **EP 0 739 451 B1**
(45) Date de publication et mention de la délivrance du brevet: **06.08.1997**
(21) Numéro de dépôt: 95904674.9
(22) Date de dépôt: 12.01.1995
(51) Int. Cl.: F04B 43/04, F04B 53/10

(54) **MICROPOMPE**
MIKROPUMPE
MICROPUMP

(30) Priorité: 14.01.1994 CH 117/94
(43) Date de publication de la demande: 30.10.1996
(73) Titulaire: WESTONBRIDGE INTERNATIONAL LIMITED, Dublin 2 (IE)
(72) Inventeur: VAN LINTEL, Harald, CH-1006 Lausanne (CH)
(74) Mandataire: Leger, Jean-François
(86) Numéro de dépôt international: IB9500028
(87) Numéro de publication internationale: WO9519502

(56) Documents cités:
- EP-A- 0 025 005
- WO-A-92/04569
- DE-A- 3 320 443
- FR-A- 2 516 606
- GB-A- 2 248 891
- SMITH 'transducers 91, digest of technical papers, IEEE catalog number 91CH2817-5' 27 Juin 1991 , IEEE , PISCATAWAY,NJ,USA cité dans la demande voir page 1049 - page 1051

## Description

La présente invention concerne une micropompe comportant au moins une plaquette de base et une seconde plaquette accolée à la plaquette de base, de manière à définir une chambre de pompage, des organes de contrôle d'entrée et de sortie en liaison directe avec la chambre de pompage, lesdits organes de contrôle étant situés sur ou dans au moins l'une des deux plaquettes, au moins une de ces plaquettes étant usinée par des techniques de micro-usinage par photolithographie d'un matériau hermétique; un volume interne (Vi) comprenant le volume de la chambre de pompage et les volumes des espaces de liaison avec lesdits organes de contrôle, la chambre de pompage comportant une paroi mobile usinée dans l'une des plaquettes, des moyens d'actionnement étant prévus pour déplacer ladite paroi mobile pour provoquer une diminution et une augmentation périodique du volume interne.

De telles pompes peuvent être utilisées notamment pour l'administration in situ de médicaments, la miniaturisation de la pompe permettant à un malade de la porter sur soi, voire éventuellement de recevoir une pompe directement implantée dans le corps. Par ailleurs, de telles pompes permettent le dosage de faibles quantités de fluide à injecter.

Dans un article intitulé "A piezoelectrtic micropump based on micromachining of silicon" paru dans "Sensors and Actuators" No 15 (1988). pages 153 à 167, H. Van Lintel et al. donnent une description de deux formes de réalisation d'une micropompe, comportant chacune un empilement de trois plaquettes, c'est-à-dire une plaquette en silicium usiné disposée entre deux plaquettes en verre.

La plaquette en silicium est gravée pour former une cavité, qui avec l'une des plaquettes en verre définit la chambre de pompage, un clapet d'entrée ou d'aspiration et au moins un clapet de sortie ou de refoulement mettant la chambre de pompage en communication respectivement avec un canal d'entrée et un canal de sortie. La partie de la plaquette formant une paroi de la chambre de pompage peut être déformée par un élément de commande constitué par exemple par une pastille ou un cristal piézoélectrique. Celle-ci est équipée de deux électrodes qui, lorsqu'elles sont raccordées à une source de tension électrique, provoquent la déformation de la pastille et, par suite, la déformation de la plaquette, ce qui provoque une variation du volume de la chambre de pompage. Cette paroi mobile ou déformable de la chambre de pompage peut ainsi être déplacée entre deux positions.

Le fonctionnement de la micropompe est le suivant. Lorsqu'aucune tension électrique n'est appliquée à la pastille piézoélectrique, les clapets d'entrée et de sortie sont en position fermée. Lorsqu'une tension électrique est appliquée, il se produit une augmentation de pression dans la chambre de pompage qui provoque l'ouverture du clapet de sortie. Le fluide contenu dans la chambre de pompage est alors refoulé vers le canal de sortie par le déplacement de la paroi déformable d'une première position vers une seconde position. Pendant cette phase, le clapet d'entrée est maintenu fermé par la pression régnant dans la chambre de pompage.

Au contraire, lorsque l'on fait décroître la tension électrique, la pression dans la chambre de pompage diminue. Ceci provoque la fermeture du clapet de refoulement et l'ouverture du clapet d'entrée. Il y a alors aspiration de fluide dans la chambre de pompage par le canal d'entrée par suite du déplacement de la paroi déformable de la seconde position vers la première position.

Le document GB-A-2.248.891 constituant l'état de la technique le plus proche révèle une micropompe du même type que celui de l'article précité. Dans cette micropompe le clapet d'entrée est cependant remplacé par un limitateur de débit présentant une section de flux restreint.

Le fonctionnement de ce type de micropompe est fortement influencé par la compressibilité du fluide contenu dans le volume interne et en fait une telle micropompe ne fonctionne pas si elle contient trop d'air; le débit pompé est ainsi diminué très fortement, voire réduit à zéro.

De même, l'amorçage de telles pompes est compliqué et requiert un appareillage important, tels que pompe à vide, enceinte d'amorçage ou dispositif d'injection. De ce fait, l'amorçage ne peut être effectué que dans un établissement spécialisé ou en usine lors de la fabrication.

Certaines micropompes sont en outre munies d'un dispositif de protection contre les surpressions à l'entrée, ce qui empêche un amorçage par l'application d'une surpression.

On connaît encore par le document DE-A-33 20 443 une pompe comportant une plaque métallique de base dans laquelle sont montées deux valves. Un élément piézoélectrique en forme de coupe constitué par une couche intérieure en Nickel et une couche extérieure en céramique est fixé par soudure sur la plaque de base. Lorsqu'une tension est appliquée à l'élément piézoélectrique, ce dernier se rétrécit de façon à expulser le liquide contenu dans la pompe. Cette pompe est d'une construction très différente que celles obtenues par micro-usinage photolithographique de matériaux hermétiques.

Le document EP-A-0.025.005 décrit une micropompe comprenant au moins une membrane flexible, de préférence métallique sur les deux côtés de laquelle sont fixés deux éléments piézoélectriques. Cette membrane est montée sur une paroi en céramique. Par application d'une tension aux éléments piézoélectriques, ces derniers se déforment pour conférer à la membrane un mouvement de pompage. Cette micropompe à membrane est également d'un type entièrement différent de celui des micropompes obtenues par micro-usinage photolithographique de matériaux hermétiques.

On connaît également de nombreux types de petites pompes qui utilisent des membranes de pompage élastiques extensibles, en particulier en élastomer, et au moyen desquelles des volumes de pompage élevés peuvent être atteints. En tant qu'exemple de ce type de pompe, citons ceux utilisés pour les aquariums. Néanmoins, ces pompes ne sont pas du type issu de procédés d'usinage tel que décrit ci-dessus. En outre, les matériaux facilement extensibles utilisés laissent passer des gaz ou des vapeurs limitant ainsi les applications à des utilisations pour lesquelles une étanchéité non hermétique est acceptable. Dans des applications dans lesquelles l'entourage de la pompe comprend des composants électroniques contenus dans une chambre étanche par exemple, aucune vapeur ne devra cependant s'échapper de la pompe.

Avec des matériaux hermétiquement étanches, comme le silicium qui est cassant et qui n'est pratiquement pas extensible, il est nécessaire de trouver d'autres solutions aux problèmes évoqués ci-dessus.

Le but de la présente invention est de créer une micropompe entièrement auto-amorçante à étanchéité complète de l'intérieur de la micropompe et qui fonctionne également de façon correcte lorsque de l'air ou un autre gaz ou fluide compressible entre dans le volume interne de la micropompe.

La micropompe est caractérisée à cet effet par le fait que l'organe de contrôle d'entrée s'ouvre vers l'extérieur de la plaquette sur ou dans laquelle il se trouve, tandis que l'organe de contrôle de sortie s'ouvre vers l'intérieur de la plaquette sur ou dans laquelle il se trouve, de sorte que la diminution du volume interne comprime un gaz contenu dans ledit volume interne à une pression suffisante pour provoquer l'ouverture de l'organe de contrôle de sortie, de façon à obtenir une micropompe auto-amorçante.

Ainsi, il est possible d'amorcer la micropompe sans appareillage particulier de façon rapide et automatique, considérant que l'air contenu dans le volume interne est évacué par l'effet de pompage même de la micropompe, tout en ayant l'avantage d'une étanchéité parfaite de ce volume interne de la micropompe.

Avantageusement, ladite diminution du volume de la chambre de pompage est comprise entre 30 et 100% dudit volume interne, de préférence plus de 50%, les volumes desdits espaces de liaisons de la chambre de pompage aux organes de contrôle d'entrée et de sortie étant inférieurs à 30% du volume interne, de préférence inférieur à 15%. On obtient ainsi un amorçage et une évacuation de l'air particulièrement efficace.

Selon un mode d'exécution préféré, la paroi mobile comprend une partie centrale rigide entourée d'une bordure élastique d'épaisseur plus faible venue d'une pièce avec la partie centrale rigide, cette dernière faisant saillie par rapport à la face de la paroi mobile qui est opposée à la chambre de pompage et étant destinée à coopérer avec lesdits moyens d'actionnement.

Une telle construction permet une évacuation très favorable de la chambre de pompage, donc un amorçage fiable. La partie centrale rigide de la paroi mobile assure un déplacement précis de cette paroi, comparable au mouvement d'un piston. Des différences de pression dans la chambre de pompage n'engendrent qu'un faible changement de volume grâce à la plus faible surface de la bordure élastique entourant la partie centrale rigide.

Favorablement la micropompe présente au moins une troisième plaquette accolée à la seconde plaquette, les moyens d'actionnement comportent un organe moteur monté de façon mobile sur la troisième plaquette, une pièce intermédiaire étant disposée entre ladite porte centrale rigide et l'organe moteur.

Cette construction donne l'avantage d'un actionnement efficace. Des variations dans la forme et dans la déformation de l'organe moteur, de préférence un élément piézoélectrique n'ont pas d'influence sur la forme de la paroi déformable.

De manière avantageuse, la pièce intermédiaire comporte une face inférieure destinée à entrer en contact avec ladite partie centrale rigide présentant une surface similaire à celle de la partie centrale rigide.

L'application du mouvement d'actionnement est effectuée sur toute la largeur de la pièce centrale rigide qui ne subit donc aucune déformation, ce qui permet une évacuation très précise de la chambre de pompage.

Selon une variante avantageuse, les organes de contrôle d'entrée et/ou de sortie du fluide sont constitués par au moins un clapet comportant deux membranes usinées dans la seconde plaquette de façon à constituer une forme en V dans la position fermée du clapet, ces membranes étant susceptibles de se séparer pour former une ouverture médiane dans la position ouverte du clapet.

Ce genre de clapet ou de valve peut être de très petite taille de sorte que l'espace reliant le clapet à la chambre de pompage présente un volume extrêmement réduit, ce qui permet de renforcer très fortement l'effet d'auto-amorçage de la micropompe.

L'invention concerne également une micropompe agencée pour l'administration de médicaments et susceptible d'être implantée dans le corps d'un patient.

D'autres avantages ressortent des caractéristiques exprimées dans les revendications dépendantes et de la description exposant ci-après l'invention plus en détails à l'aide de dessins qui représentent schématiquement et à titre d'exemple quatre modes d'exécution.

La figure 1 illustre un premier mode d'exécution de l'invention en coupe selon la ligne I-I de la figure 2.

La figure 2 est une vue en coupe horizontale selon la ligne II-II de la figure 1.

La figure 3 représente un second mode d'exécution en coupe.

La figure 4 représente un troisième mode d'exécution en coupe.

Les figures 5a) à 5d) illustrent un type particulier de clapet utilisé, représenté en coupes transversales et en vues de dessous pour les positions ouvertes et fermées.

La figure 6 représente un quatrième mode d'exécution en coupe selon la ligne VIII-VIII de la figure 7.

La figure 7 est une vue en coupe horizontale selon la ligne IX-IX de la figure 6.

Les figures 8a et 8b représentent un autre type particulier de clapet utilisé en coupe transversale et en vue en plan.

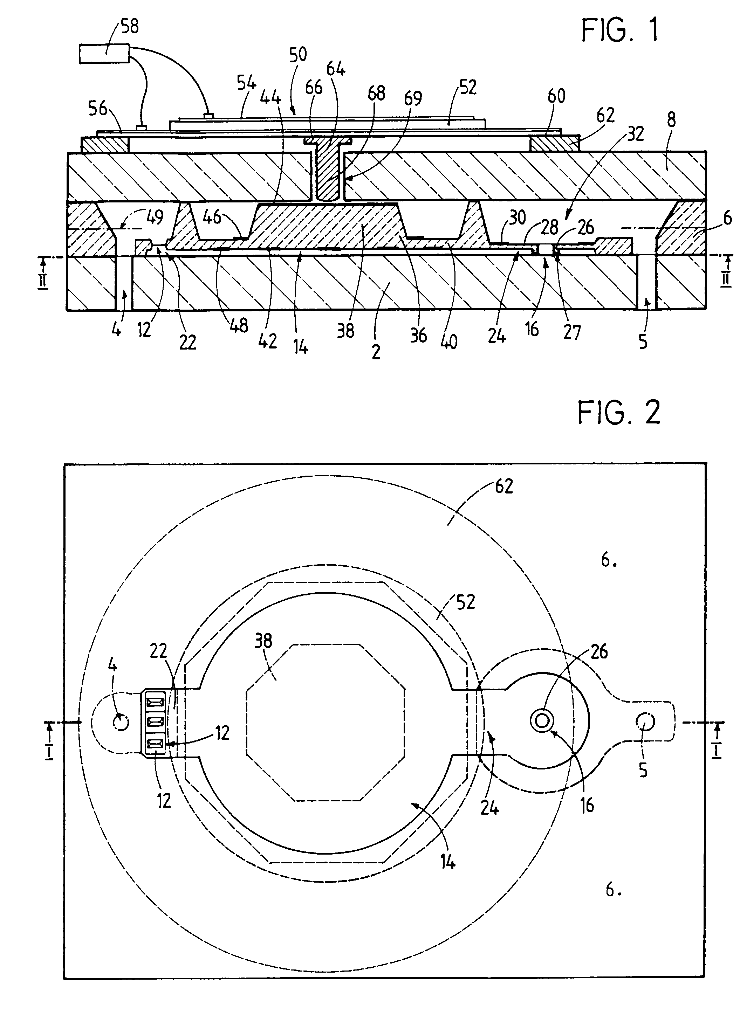

Sur les figures 1 à 4, 6 et 7, un même élément représenté sur plusieurs figures est désigné sur chacune de celles-ci par la même référence numérique. Dans les modes d'exécution qui vont être décrits, la micropompe est équipée d'un ou plusieurs clapet d'entrée ou d'un limitateur de débit et d'un clapet de sortie. Il convient de noter toutefois que l'invention s'applique également à des micropompes comportant plusieurs clapets disposés entre la chambre de pompage et la sortie. La micropompe peut également être munie d'une pluralité de sorties. Les clapets d'entrée et de sortie pourront être remplacés par tout autre organe de contrôle d'entrée ou de sortie du fluide, tel que des limitateurs de débit.

Il est à noter que, par souci de clarté, les épaisseurs de diverses plaquettes composant la micropompe ont été fortement exagérées sur les dessins.

En référence aux figures 1 et 2, la micropompe selon le premier mode d'exécution comporte une plaquette de base 2, en verre de préférence. Cette plaquette de base 2 est percée de deux canaux 4, 5 formant les conduits d'entrée et de sortie de la pompe.

Le conduit d'entrée 4 peut être raccordé à un réservoir non illustré dans lequel se trouve la substance liquide à pomper, par exemple un médicament à administrer avec un dosage précis. Dans cette application, la micropompe peut être portée sur le corps du patient, voire être implantée. Le conduit de sortie 5 peut être branché à une aiguille d'injection (non représentée) par exemple.

La plaquette de base 2 est surmontée d'une plaquette intermédiaire 6 en silicium ou en un autre matériau usinable par gravure à l'aide de techniques photolithographiques. Elle est accolée à la plaquette de base 2 par des techniques de liaison connues, telle que la technique connue sous le terme anglais "anodic bonding" ou soudure anodique comportant un échauffement à environ 300°C et l'application d'une différence de potentiel d'environ 500V entre les plaquettes.

Une plaquette supérieure 8, de préférence en verre, est accolée par les mêmes techniques à la plaquette intermédiaire 6.

A titre d'exemple, la plaquette intermédiaire 6 en silicium peut avoir une orientation cristalline <100>, afin de se prêter avec succès à la gravure. Les plaquettes 2, 6 et 8 sont de préférence soigneusement polies. Ces plaquettes 2, 6, et 8 sont ensuite avantageusement rendues hydrophiles, en particulier dans le cas où la substance utilisée dans la micropompe est une solution aqueuse. La plaquette en silicium 6 peut à cette fin être plongée dans du HNO₃ bouillant.

Pour fixer les idées, les épaisseurs des plaquettes 2, 6 et 8 peuvent respectivement être d'environ 1mm, 0,3mm et 0,8mm pour une dimension en surface des plaquettes de l'ordre de 15 par 20 mm.

Les conduits d'entrée ou d'aspiration 4 et de sortie ou de refoulement 5 sont principalement reliés par trois clapets d'entrée 12, une chambre de pompage 14 et un clapet de sortie 16.

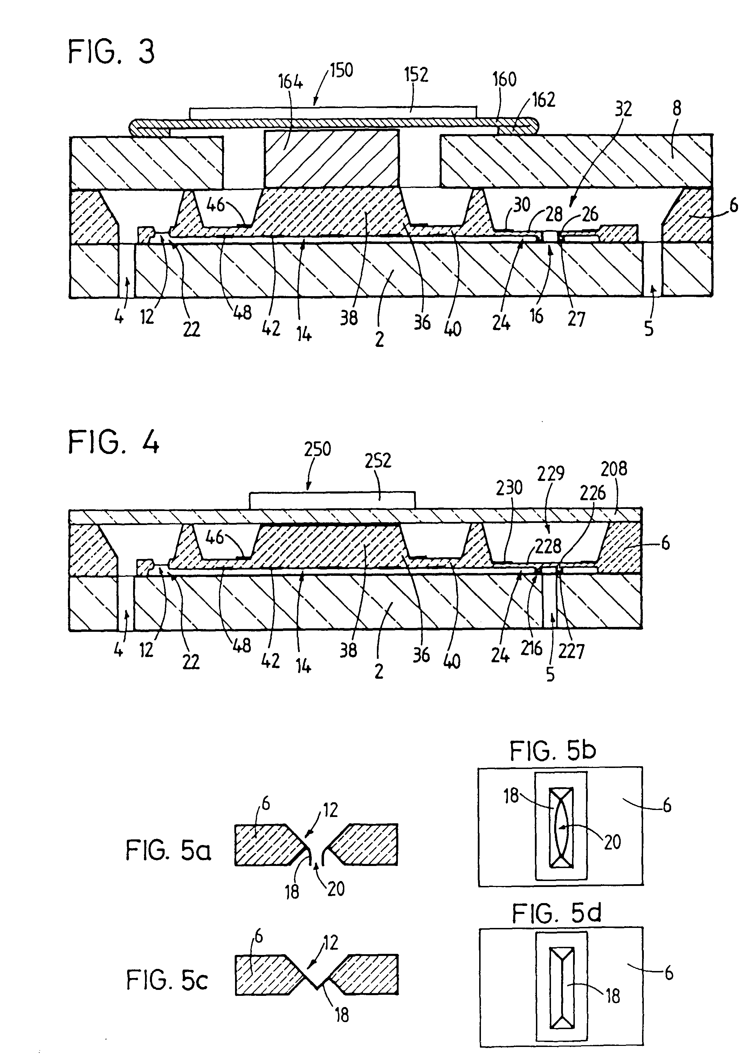

Les clapets d'entrée 12 sont illustrés plus particulièrement en référence aux figues Sa) à 5d) et comportent chacune deux membranes 18 usinées dans la plaquette en silicium 6 de façon à constituer une forme en V dans la position fermée du clapet (fig. 5c) et d)). Les membranes 18 sont susceptibles de se séparer à leur jonction pour former une ouverture médiane 20 dans la position ouverte du clapet (fig. 5a) et b)). Ce type de valve ou clapet obtenu par micro-usinage est plus particulièrement décrit par L. Smith and B. Hök dans un article intitulé "A silicon self-aligned non reverse valve" paru dans "Transducers 91, Digest of Technical papers, pp. 1049.1051 IEEE catalog number 91CH2817-5, IEEE, piscataway, NJ (1991).

Ces valves permettent une construction de très petite taille et ne nécessitent qu'un espace de liaison 22 de très faible volume pour le raccord à la chambre de pompage. Le phénomène de microcavitation qui apparaît dans les valves du type habituel présentant un clapet coopérant avec un siège est évité. En effet, dans ces valves du type habituel, une forte diminution de la pression est observée très localement au niveau du siège de valve au moment de la séparation du clapet de son siège. Cette diminution de pression peut donner naissance à la formation de microbulles de gaz, lorsque la pression à cet endroit du liquide devient inférieure à la tension de vapeur de celui-ci. Cet inconvénient ne peut pas apparaître avec le présent type de clapet 12 à membranes en V du fait que l'écartement des deux membranes se fait de manière glissante avec écoulement du fluide le long des deux membranes.

Du fait de la dimension très faible des trois clapets, l'espace de liaison 22 situé sous les trois clapets d'entrée 12 présente un volume très restreint et se raccorde immédiatement à la chambre de pompage 14.

Cette dernière est reliée au clapet de sortie 16 par un second espace de liaison 24 qui entoure la nervure annulaire 26 de ce clapet de sortie 16. Ce second espace de liaison 24 est de préférence également d'un volume aussi restreint que possible.

Le clapet de sortie 16 de type conventionnel est de même usiné dans la plaquette en silicium 6 et comporte une membrane 28 portant la nervure annulaire 26 revêtue d'une couche d'oxyde 27 conférant à la membrane 28 une précontrainte sollicitant le sommet de la nervure 26 contre la plaquette inférieure 2 qui sert de siège de clapet. Des couches d'oxyde 30 appliquées de l'autre côté de la membrane 28 renforcent cette précontrainte. La nervure 26 délimite un compartiment 32 extérieur à la nervure 26 communiquant avec le conduit de sortie 5.

Il est bien entendu que l'on pourra également prévoir d'autres types d'organes de contrôle de sortie du fluide, tels que des clapets du type à membrane en V ou par exemple des limitateurs de débit.

La chambre de pompage 14 est de forme sensiblement circulaire. Son volume est modulé par une membrane de pompage 36 constituant une paroi mobile ou déformable de la chambre de pompage 14. Cette paroi mobile est usinée dans la plaquette de silicium 6 et comporte une partie centrale rigide 38 relativement large par rapport à la largeur totale de la membrane de pompage 36. Le diamètre de cette partie centrale 38 varie entre 20 et 90 % du diamètre de la membrane de pompage 36, de préférence entre 50 et 80 %. Cette partie centrale rigide 38 comporte une épaisseur nettement plus grande que le bord annulaire 40 de la membrane de pompage. Pour fixer les idées, le bord 40 présente une épaisseur entre 10 et 100µm, tandis que la partie centrale rigide 38 présente une épaisseur qui et de 10 à 50µm inférieure à l'épaisseur totale de la plaquette 6, ce qui donne par exemple une épaisseur de 300 µm.

Cette membrane de pompage 36 comporte sur sa surface inférieure en regard de la plaquette 2 des zones 42 munies d'une fine couche d'oxyde de silicium permettant d'éviter un collage de la membrane 36 à la plaquette 2. Une fine couche d'oxyde de silicium 44 similaire est prévue sur la surface supérieure de la partie centrale rigide 38 dans un but identique. Des couches d'oxyde de silicium 46, 48 appliquées des deux côtés du bord annulaire 40 sont destinées à conférer à la membrane une certaine précontrainte (non visible) vers le haut à la figure 1. La partie centrale rigide 38 et la plaquette supérieure 8 avec laquelle elle coopère constituent des éléments de butée limitant le mouvement d'aspiration de la membrane de pompage 36.

Il est à noter que les clapets ou valves d'entrée 12 et de sortie 16 sont tous disposés du même côté d'une surface fictive médiane 49 de la plaquette 6; la chambre de pompage 14 se trouve du même côté de cette surface médiane. Le clapet de sortie 16, qui présente une construction différente de celle des clapets d'entrée 12, s'ouvre en direction de cette surface médiane 49 tandis que les clapets d'entrée 12 s'ouvrent dans la direction opposée vers le bas en s'éloignant de cette surface médiane.

Un dispositif d'actionnement 50 de la membrane de pompage 36 comporte un organe moteur sous forme d'un élément piézoélectrique 52 pourvu d'électrodes 54, 56 branchés à un générateur 58 destiné à fournir une tension alternative. Cet élément peut être celui commercialisé par la société Philips sous la dénomination PXE-52. Il est fixé par tous moyens adéquats tels que collage ou soudure, sur une lame élastique 60 en métal, silicium ou en matière plastique. Cette lame 60 est montée par l'intermédiaire d'un élément d'espacement 62 sur la plaquette supérieure 8. Cet élément d'espacement 62 pourra être constitué par un anneau de support en matière plastique, métallique ou silicium. Il pourrait également être formé par une épaisseur prédéterminée de colle ou par du verre venu d'une pièce avec la plaquette 8. Une pièce intermédiaire 64 en forme de punaise peut être rendue solidaire par sa tête plate 66 par tous moyens adéquats, tel que collage ou soudure, de la lame élastique 62. Elle agit sur la partie centrale rigide 38 de la membrane de pompage 36 grâce à sa tige verticale 68 traversant la plaquette supérieure par un perçage 69. Il peut par ailleurs exister un faible jeu ou une contrainte mécanique entre la tige verticale 68 et la membrane de pompage 36, lorsque la pompe est au repos.

Le dispositif d'actionnement 50 comprenant un élément piézoélectrique 52 et une lame élastique 60, pourra également être remplacé par un dispositif comprenant deux ou plusieurs plaquettes piézoélectriques accolées ou des disques piézocéramiques et métalliques combinés.

Ainsi, l'élément piézoélectrique 52 est indépendant de la membrane de pompage 36. Des effets d'hystérésis de l'élément piézoélectrique 52 ("piézocreep") ou des variations ou déteriorations de cet élément n'ont pas d'influence sur la forme de la membrane de pompage 36 considérant que cette dernière est indépendante de l'élément piézoélectrique 52 et mise en mouvement grâce à la pièce intermédiaire 64. Cette construction permet d'obtenir un grand volume de fluide déplacé pour un diamètre donné de la membrane de pompage, considérant que la partie centrale rigide 38 agit à la manière d'un piston. Les parties usinées de la micropompe peuvent être davantage miniaturisées tout en conservant un dispositif d'actionnement d'une taille quelconque, relativement grande. Cette miniaturisation des parties usinées permet d'abaisser les coûts de revient.

La micropompe décrite comporte un volume interne (Vi) comprenant le volume (Vp) de la chambre de pompage 14, le volume (Ve) de l'espace de liaison 22 délimité d'un côté par les clapets d'entrée 12 et relié de l'autre côté à la chambre de pompage 14, et le volume (Vs) de l'espace de liaison 24 vers le clapet de sortie, y compris sa partie annulaire entourant la nervure annulaire 26. Les moyens d'actionnement 50 déplacent la paroi mobile 26 pour provoquer une variation périodique (ΔVp), diminution puis augmentation du volume (Vp) de la chambre de pompage 14. La diminution du volume interne (Vi) de la micropompe due à la diminution du volume (Vp) de la chambre de pompage est telle qu'un gaz, par exemple de l'air, ou tout autre fluide compressible contenu dans le volume interne de la micropompe est comprimé au moins à une pression suffisante pour provoquer l'ouverture du clapet de sortie 16 de façon que cet air puisse être chassé de la micropompe qui est alors auto-amorçante.

Pour obtenir cet effet, il est nécessaire que la variation ou diminution (ΔVp) du volume de la chambre de pompage 14, provoquée par le mouvement de la paroi mobile 36 ne soit pas beaucoup plus faible que le volume (Vp) de la chambre de pompage. En outre, le volume (Ve et Vs) des espaces de liaison 22 et 24 doit être aussi faible que possible, ce qui peut être obtenu par micro-usinage.

Avantageusement, la variation ou diminution (ΔVp) est comprise entre 30 et 100 % du volume interne (Vi), de préférence plus de 50 %.

Le volume (Ve et Vs) des deux espaces de liaison 22 et 24 ensemble est avantageusement inférieur à 30 % du volume interne (Vi) et de préférence inférieur à 15 % de ce volume interne.

Pour fixer les idées, le mode d'exécution pourra présenter une paroi mobile 36 d'un diamètre de 7 mm avec une partie centrale rigide d'un diamètre de 5 mm et un déplacement vertical de 10 micromètres. La variation ou diminution du volume (ΔVp) de la chambre de pompage 14 sera alors approximativement de 0.28 mm³. Le volume (Vp) de la chambre de pompage étant de 0,38 mm³, celui (Ve) de l'espace de liaison vers les clapets d'entrée 22 de 0,015 mm³, celui (Vs) de l'espace de liaison 24 vers le clapet de sortie de 0,03 mm³. Le volume interne (Vi) sera approximativement de 0,43 mm³.

Ainsi la variation ou diminution (ΔVp) sera d'environ 65 % du volume interne (Vi) et le volume (Ve + Vs) des deux espaces de liaison 22, 24 ensemble ne sera que de 10% environ du volume interne (Vi) de la micropompe.

En admettant que la micropompe au repos, non amorcée, est remplie d'air à une pression d'un bar et que l'on comprime ensuite cet air à un volume de 35 % du volume à l'origine, une pression supérieure à 2,5 bar est générée dans le volume interne (Vi) de la micropompe.

Etant donné qu'une pression interne de 1,5 bar est nécessaire pour provoquer l'ouverture du clapet de sortie 16, la pression générée à l'intérieur de la micropompe est largement suffisante pour rendre cette dernière entièrement auto-amorçante. Grâce à la pièce centrale rigide 38 d'un diamètre large fonctionnant à la manière d'un piston et aux espaces de liaison 22 et 24 très petits, il est possible d'obtenir des pressions suffisantes à l'intérieur de la micropompe pour permettre un auto-amorçage.

Ceci n'est pas le cas dans les micropompes connues qui comportent généralement un espace d'un volume assez considérable du côté intérieur du clapet d'entrée. Ainsi, le volume interne est généralement de l'ordre de 3 mm³, tandis que la variation de volume due au mouvement de pompage est d'environ 0,1 mm³ avec une membrane de pompage classique d'un diamètre de 7 mm. Une telle micropompe ne peut en l'occurence pas fonctionner dès qu'un gaz remplit partiellement on entièrement son volume interne. Cet inconvénient majeur du fonctionnement et de l'amorçage est entièrement évité grâce à la présente invention. De même, lors de la montée complète de la membrane de pompage 36, l'augmentation du volume interne (Vi) de la micropompe due à l'augmentation du volume (Vp) de la chambre de pompage 14 est telle que le gaz, à savoir l'air, restant dans le volume interne de la micropompe après la fermeture du clapet de sortie 16 est décomprimé à une pression suffisamment basse pour provoquer l'ouverture du ou des clapets d'entrée 12.

La pièce centrale rigide 36, plus épaisse, destinée à entrer en contact avec la plaquette supérieure 8, forme un élément de butée opposé aux éléments de butée constitués par les zones à oxydes de silicium 42 entrant en contact avec la plaquette de base 2.

Les mouvements d'aspiration et d'expulsion de la membrane de pompage 36 sont ainsi mécaniquement contrôlés du côté supérieur et inférieur. Ceci permet d'obtenir une quantité de substance pompée très précise à chaque aller-retour de la membrane. La partie centrale rigide 38 est comparable à un piston dont le mouvement est bien défini. Etant donné que le bord annulaire 40 de la membrane de pompage 36 présente une surface relativement petite par rapport à la surface totale de la membrane de pompage 36, des différences de pression dans la chambre de pompage 14 n'engendrent que de faibles changements de volume sous la membrane de pompage 36.

En outre, les zones à oxyde 42 évitent un effet de collage de la membrane de pompage 36 pendant le soudage anodique ou un effet de succion de cette membrane, lorsque celle-ci se déplace de sa position la plus basse vers le haut.

Des contacts électriques ou électrodes non illustrés peuvent être disposés en regard l'un de l'autre sur la partie centrale rigide 38 et sur la surface inférieure de la plaquette supérieure 8. Ces contacts sont alors prolongés vers l'extérieur de la pompe et connectés à un circuit électrique non illustré permettant de contrôler le fonctionnement de la membrane de pompage 36 et l'aspiration du fluide. Des circuits adéquats sont par exemple décrits dans la demande de brevet européen No 0.498.863.

Le mode général de fonctionnement de cette pompe est semblable à celui décrit dans l'article de H. Van Lintel et al. intitulé : " A piezoelectric micropump based on micromachining of silicon" paru dans "Sensor and Actuators" No 15 (1988) pages 153 à 167.

Par rapport à ce type de micropompe connu, la micropompe conformément à la présente invention permet donc d'obtenir un auto-amorçage sûr et fiable. En outre, la micropompe présente un dosage très précis à chaque mouvement alternatif, un dosage qui est pratiquement indépendant de la pression régnant dans les conduits d'entrée et de sortie, de la performance de l'élément piézoélectrique et des déteriorations et phénomènes d'hystérésis connus pour ce genre de dispositif d'actionnement. En outre, le mouvement de la membrane de pompage est contrôlé de façon précise autant par la pièce intermédiaire rigide 38 que les zones à oxyde 42. Le débit est donc défini par les caractéristiques d'usinage de la membrane de pompage 36 et par la fréquence du dispositif d'actionnement.

Ce type de pompe permet l'utilisation d'éléments piézoélectriques possédant des variations assez larges dans leurs caractéristiques. En outre, il n'est pas nécessaire de calibrer les pompes pour chaque élément utilisé.

Du fait de la fixation extérieure de l'élément, ce dernier peut être aisément remplacé en cas de défectuosité.

Il est bien entendu que le mode d'exécution décrit ci-dessus ne présente aucun caractère limitatif et qu'il peut recevoir toutes modifications désirables à l'intérieur du cadre tel que défini par la revendication 1. En particulier, la construction des clapets et l'agencement des clapets et des conduits de sortie et d'entrée, ainsi que de la chambre de pompage pourra être très différent. Le clapet de sortie pourra également être de type à deux membranes en V. La chambre de pompage et ces clapets ou organes de contrôle du fluide pourront également être disposés en partie ou dans leur totalité sur la plaquette de base, si tel semble préférable. Les clapets ou organes de contrôle sont alors disposés sur le côté de la plaquette sur laquelle ils sont usinés qui est le plus proche de la chambre de pompage, les clapets d'entrée 12 s'ouvrant vers l'extérieur de la plaquette sur laquelle ils se trouvent, tandis que les clapets de sortie 16 s'ouvrent vers l'intérieur de la plaquette sur laquelle ils se trouvent. La distribution des zones à oxyde pourra être adaptée aux précontraintes souhaitées pour les clapets et le pompage. Le dispositif d'actionnement pourra présenter un organe moteur d'un autre type qu'un élément piézoélectrique.

La pièce intermédiaire 64 pourrait être venue d'une pièce avec la lame élastique 60 ou encore avec l'élément piézoélectrique. Elle pourrait également être librement disposée entre la lame élastique et la membrane de pompage.

La pompe pourra en outre présenter un ou plusieurs vis traversant la plaquette supérieure 8 et coopérant par leur extrémité avec la partie centrale rigide 38. Ces vis constituent ainsi des éléments de butées réglables permettant d'ajuster l'amplitude de mouvement d'aspiration.

Des vis de réglage pourront également être montées sur la lame 60. En outre, il serait possible de monter des vis de réglage dans la tête plate 66 de la pièce intermédiaire.

Le deuxième mode d'exécution illustré à la figure 3 diffère du premier mode d'exécution uniquement par la constitution du dispositif d'actionnement. De ce fait, des éléments analogues aux deux modes d'exécution portent les mêmes chiffres de référence et ne seront plus décrits en détails.

Ce second mode d'exécution comporte également une plaquette de base 2 percée de conduits d'entrée 4 et de sortie 5 et une plaquette supérieure 8. Entre ces plaquettes 2 et 8 est intercalée la plaquette intermédiaire 6 en silicium usinée par des techniques photolitographiques pour obtenir un ou plusieurs clapets d'entrée 12 et de sortie 16 et une chambre de pompage 14.

Des fines couches d'oxyde 27, 30, 46, 48 permettent d'obtenir des précontraintes prédéterminées dans la membrane en silicium usinée.

La chambre de pompage 14 est de forme sensiblement circulaire et reliée par deux espaces de liaison 22, 24 aux clapets d'entrée et de sortie. Usinée dans la plaquette de silicium 6, la membrane de pompage 36 sous forme d'une paroi mobile, déformable comprend une partie centrale rigide 38 plus épaisse.

Le dispositif d'actionnement 150 présente une lame métallique élastique 160 présentant des bords recourbés 162 formant des éléments d'espacement collés à la surface supérieure de la plaquette 8. Un organe moteur sous forme d'un élément piézoélectrique 152 est fixé par soudure ou collage à la lame 160. Il agit grâce à une pièce intermédiaire 164 sur la partie centrale rigide 38 de la membrane de pompage 36. Cette pièce intermédiaire 164 présente une section droite et une surface inférieure similaire à celle de la partie centrale rigide 38 et est fixée à cette dernière par tous moyens adéquats, tel que la soudure anodique ou le collage. Grâce à cette pièce intermédiaire 164 d'un diamètre considérable, la membrane de pompage 36 est déplacée verticalement sans provoquer de déformation sur la plus grande partie de sa surface. L'évacuation de la chambre de pompage 14 est donc particulièrement efficace, ce qui renforce encore davantage l'effet d'auto-amorçage de la microvalve pour une géométrie donnée du volume interne (Vi). En outre, la construction et le montage sont simples, ce qui permet de baisser le prix de revient de la micropompe.

Le troisième mode d'exécution représenté à la figure 4 diffère du premier et second mode d'exécution principalement par la constitution du clapet de sortie 216 et du dispositif d'actionnement 250. De ce fait, des éléments analogues aux trois modes d'exécution portent les mêmes chiffres de référence et ne seront plus décrits de façon détaillée.

Ce troisième mode d'exécution comporte également une plaquette de base 2 munie de conduit d'entrée 4 et de sortie 5 et une plaquette supérieure 8. Entre ces plaquettes 2 et 8 est intercalée la plaquette intermédiaire 6 en silicium usinée par des techniques photolitographiques pour obtenir un ou plusieurs clapets d'entrée 12 et de sortie 216 et une chambre de pompage 14. Des fines couches d'oxyde 227, 230, 46 et 48 permettent d'obtenir des précontraintes prédéterminées dans la membrane en silicium usinée.

La chambre de pompage est également de forme circulaire et reliée par des espaces de liaison 22 et 24 aux clapets d'entrée et de sortie.

La plaquette supérieure 208 en verre est dans ce mode d'exécution d'une épaisseur faible de l'ordre de 0,2 mm de façon à pouvoir être déformée élastiquement par l'élément piézoélectrique 252, qui est fixé par collage à sa surface supérieure. L'élément piézoélectrique 252 agit par déformation élastique de la plaquette en verre sur la partie centrale rigide 38 pour obtenir le mouvement de pompage.

Cette construction ne nécessite pas de perçage de la plaquette en verre, donc un montage plus rapide et aisé.

Le clapet de sortie 216 comporte une membrane non percée 228 présentant une nervure annulaire 226 disposée autour du canal de sortie 5 et recouvert d'une fine couche d'oxyde de silicium 227.

Ce mode d'exécution comprend en outre une chambre 229 disposée entre la membrane 228 et la plaquette supérieure 208. Cette chambre 229 pourra être reliée au conduit d'entrée 4 et constituer une protection contre des surpressions à l'entrée de la microvalve fermant le conduit de sortie 5 en cas de surpression. Elle pourra également être reliée à des organes de détection du fonctionnement de la valve. Finalement, elle pourra aussi être scellée ou reliée à l'air ambiant. Cette construction nécessite un très bon alignement entre la nervure annulaire 226 et le conduit de sortie 5 qui doit présenter un faible diamètre.

Au lieu d'un clapet d'entrée, les micropompes décrites pourraient présenter en tant qu'organe de contrôle du fluide d'entrée, un limitateur de débit formé par un conduit de très faible section reliant le conduit d'entrée directement à la chambre de pompage. L'espace de liaison entre ces deux éléments présente donc ici un très faible volume, ce qui est particulièrement favorable pour l'auto-amorçage.

Le dispositif d'actionnement constitué par un élément piézoélectrique pourrait également être collé directement sur la membrane de pompage et connecté à un générateur destiné à fournir une tension alternative.

La fréquence d'actionnement de l'élément piézoélectrique serait dans cette variante à limitateur de débit plus élevé, à savoir entre 10 et 100 Hertz au lieu de 1 à 10 Hertz pour les modes d'exécution à clapets d'entrée. Considérant cette fréquence plus élevée et la faible section de flux du limitateur de débit, il s'installe un courant d'auto-amorçage susceptible d'évacuer le gaz contenu dans le volume interne (Vi) de la micropompe vers le conduit de sortie. Il est bien entendu que dans cette variante également, la variation (ΔVp) du volume de la chambre de pompage 14 ne doit pas être beaucoup plus faible que le volume (Vp) de la chambre de pompage et le volume interne (Vi) de la micropompe. Les volumes (Ve et Vs) des espaces de liaison vers le limitateur et vers le clapet de sortie doivent être aussi faibles que possible pour obtenir un auto-amorçage efficace.

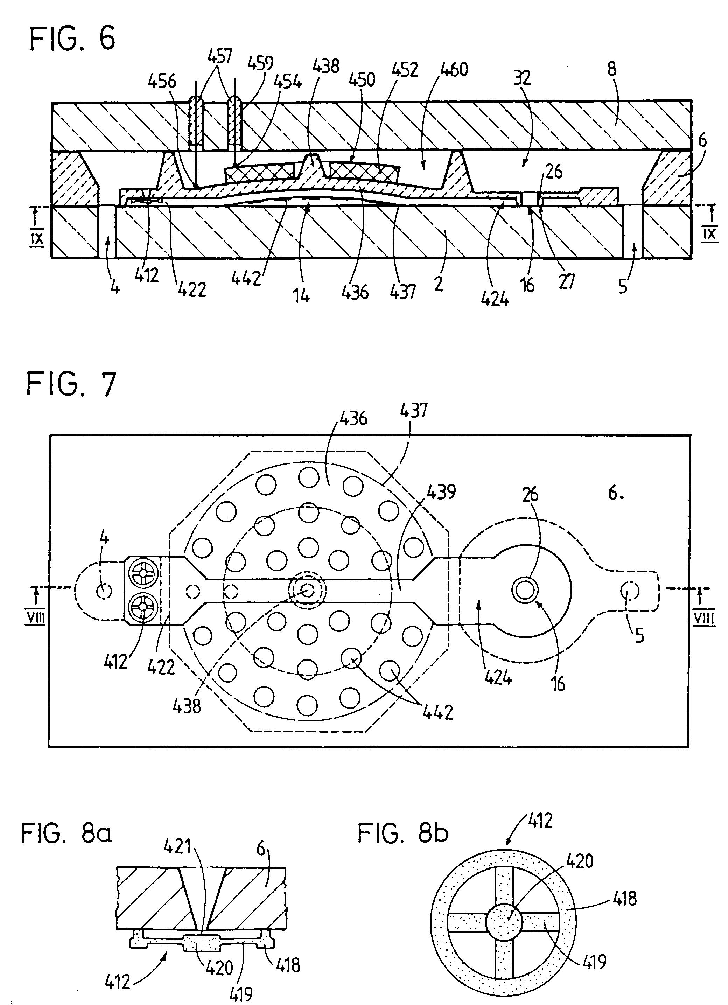

Le quatrième mode d'exécution représenté aux figures 6 à 8 comprend, comme le premier mode d'exécution, une plaquette de base 2 en verre avec des conduits d'entrée et de sortie 5, une plaquette intermédiaire 6 en silicium usiné par gravure et une plaquette supérieure 8 de préférence en verre. Ce mode d'exécution est muni de deux valves d'entrée 412 qui sont du type décrit dans l'article de Shoji Sh. et al., Technical Digest of the 7th Sensor Symposium, 1988, pages 217 à 220. En référence à la figure 8, ces valves en silicium polycristallin sont obtenues par des techniques de microusinage de surface. Elles comportent une structure annulaire 418 accolée à la plaquette 6 et reliée par quatre bras 419 à une pièce centrale d'obturation 420 destinée à coopérer avec un trou 421 prévu dans la plaquette 6.

La valve de sortie 16 est du même type que celle décrit en référence à la figure 1. La membrane de pompage 436 présente ici cependant dans la position de repos une forme bombée vers le haut et comporte une partie centrale 438 de plus petit diamètre. Sa surface inférieure dirigée vers la chambre de pompage 14 est munie d'une pluralité de zones circulaires 442 à oxyde de silicium destinées à prévenir un collage ou une succion de la membrane 436. La ligne interrompue 437 indique la limite du contact entre la membrane 436 et la plaquette de base 2. Cette membrane 436 comporte en outre une creusure 439 reliant l'espace de liaison 422 à l'espace de liaison 424. Cette creusure 439 est également destinée à éviter une succion de la membrane 436. Elle pourrait bien entendu également être prévue dans la plaquette de base 2.

Le dispositif d'actionnement 450 comprend un élément piézoélectrique 452 de forme annulaire accolé à la membrane 436 autour de sa partie centrale 438. Deux électrodes 454 et 456 sont connectées par des fils 457 à un générateur de tension alternative. Les ouvertures 459 dans la plaquette 8 sont fermées de façon hermétique par exemple au moyen d'une colle epoxy ou d'une soudure.

Lorsque l'élément piézoélectrique 452 est activé, la membrane 436 se déplace vers le bas à la figure 6 pour présenter une forme quasi plate, ce qui permet d'obtenir un excellent rapport de compression de la chambre de pompage 14 qui présente alors un volume résiduel très faible. La hauteur du bombage de la membrane de pompage 436 correspond donc sensiblement au déplacement de pompage de cette membrane.

Cette forme bombée de la membrane 436 peut être obtenue en mettant sous sous-pression la chambre 460 située au-dessus de la membrane hermétiquement fermée. Elle pourrait également être obtenue en appliquant sur sa surface supérieure une couche d'oxyde induisant une pré-contrainte de déformation adéquate.

Il est à noter qu'une membrane ainsi bombée vers le haut pourrait également être très avantageuse dans les autres modes d'exécution décrits précédemment. De même, les valves 412 à silicium polycristallin pourrait également être utilisées dans le cadre des autres modes d'exécution et en tant que valve de sortie. Le microusinage de ces valves pourrait également être obtenu avec d'autres matériaux, tels que le nitrure de silicium ou des métaux utilisés en galvanoplastie. La pièce centrale 438 servant de butée d'ouverture pourrait également manquer, mais dans le cas présent elle est utile pour augmenter la précision de pompage et en tant qu'élément de sécurité pour éviter une rupture de la membrane de pompage 436 lors de surpression dans le canal d'entrée 4.

Ainsi, les modes d'exécution décrits permettent, grâce à un effet d'auto-amorçage, d'évacuer l'air entrant dans la pompe en observant uniquement une diminution temporaire du débit de pompage jusqu'à ce que l'ensemble de l'air ait été entraîné vers le conduit de sortie de la micropompe.

Les modes d'exécution décrits sont particulièrement adaptés pour l'administration de médicaments et pour une implantation dans le corps d'un patient.

## Revendications

1. Micropompe comportant au moins une plaquette de base (2) et une seconde plaquette (6) accolée à la plaquette de base, de manière à définir une chambre de pompage (14), des organes de contrôle d'entrée (12) et de sortie (16) en liaison directe avec la chambre de pompage, lesdits organes de contrôle étant situés sur ou dans au moins l'une des deux plaquettes, au moins une de ces plaquettes étant usinée par des techniques de micro-usinage par photolithographie d'un matériau hermétique; un volume interne (Vi) comprenant le volume de la chambre de pompage (14) et les volumes des espaces de liaison (22,24) avec lesdits organes de contrôle, la chambre de pompage (14) comportant une paroi mobile (36) usinée dans l'une des plaquettes, des moyens d'actionnement (50) étant prévus pour déplacer ladite paroi mobile (36) pour provoquer une diminution et une augmentation périodique de volume interne, caractérisée en ce que l'organe de contrôle d'entrée (12) s'ouvre vers l'extérieur de la plaquette sur ou dans laquelle il se trouve, tandis que l'organe de contrôle de sortie (16) s'ouvre vers l'intérieur de la plaquette sur ou dans laquelle il se trouve, de sorte que la diminution du volume interne comprime un gaz contenu dans ledit volume interne à une pression suffisante pour provoquer l'ouverture de l'organe de contrôle de sortie, de façon à obtenir une micropompe auto-amorçante.

2. Micropompe selon la revendication 1, caractérisée en ce que la chambre de pompage (14) et les espaces de liaison (22, 24) sont conformés de façon que l'augmentation du volume interne de la micropompe due à l'augmentation du volume de la chambre de pompage est telle que le gaz restant dans la micropompe après la fermeture de l'organe de contrôle de sortie (16) du fluide est décomprimé à une pression suffisamment basse pour provoquer l'ouverture de l'organe de contrôle d'entrée du fluide (12).

3. Micropompe selon la revendication 1 ou 2, caractérisée en ce que la diminution du volume (ΔVp) de la chambre de pompage est comprise entre 30 et 100% dudit volume interne (Vi), de préférence plus de 50%, les volumes (Ve et Vs) desdits espaces de liaisons de la chambre de pompage (14) aux organes de contrôle d'entrée (12) et de sortie (16) étant inférieurs à 30% du volume interne (Vi), de préférence inférieur à 15%.

4. Micropompe selon la revendication 1, caractérisée en ce que la chambre de pompage (14) est disposée d'un côté d'une surface médiane de la plaquette dans ou sur laquelle est est usinée, chacun des organes de contrôle d'entrée (12) et de sortie (16) étant disposé dans ou sur le côté de la plaquette sur laquelle il est usiné qui est le plus proche de la chambre de pompage.

5. Micropompe selon l'une des revendications précédentes, caractérisée en ce que le ou les organes d'entrée (12) présentent une construction différente de celle du ou des organes de sortie (16).

6. Micropompe selon l'une des revendications précédentes, caractérisée en ce que la paroi mobile (36) comprend une partie centrale rigide (38) entourée d'une bordure élastique (40) d'épaisseur plus faible venue d'une pièce avec la partie centrale rigide (38), cette dernière faisant saillie par rapport à la face de la paroi mobile qui est opposée à la chambre de pompage (14) et étant destinée à coopérer avec lesdits moyens d'actionnement (50).

7. Micropompe selon la revendication 6, caractérisée en ce que la largeur de ladite partie centrale rigide (38) varie entre 20 et 90 % de la largeur de la paroi mobile (36), de préférence entre 50 et 80 %.

8. Micropompe selon l'une des revendications précédentes, caractérisée en ce qu'elle présente au moins une troisième plaquette (8) accolée à la seconde plaquette (6), les moyens d'actionnement (50) comportent un organe moteur (52) monté de façon mobile sur la troisième plaquette, une pièce intermédiaire (64) étant disposée entre ladite partie centrale rigide (38) et l'organe moteur (52).

9. Micropompe selon la revendication 8, caractérisée en ce que l'organe moteur (52) est monté de façon mobile sur la face extérieure de ladite troisième plaquette (8), ladite pièce intermédiaire (64) traversant cette troisième plaquette (8) par une ouverture (69).

10. Micropompe selon la revendication 9, caractérisée en ce que l'organe moteur est un élément piézoélectrique (52) qui est monté par l'intermédiaire d'un élément d'espacement (62) sur la face extérieure de la troisième plaquette (8).

11. Micropompe selon l'une des revendications 8 à 10, caractérisée en ce que la pièce intermédiaire (64) comprend une tête plate (66) solidaire de l'organe moteur (52) et une tige (68) traversant la troisième plaquette (8) et agissant par son extrémité sur la paroi mobile (36).

12. Micropompe selon la revendication 8, caractérisée en ce que la pièce intermédiaire (164) comporte une face inférieure destinée à entrer en contact avec ladite partie centrale rigide (38) présentant une surface similaire à celle de la partie centrale rigide (38).

13. Micropompe selon la revendication 6, caractérisée en ce qu'elle comporte une troisième plaquette (208) présentant une épaisseur telle qu'elle puisse être déformée élastiquement par le dispositif d'actionnement (250) qui est monté sur la face extérieure de la troisième plaquette (208), la face intérieure de cette troisième plaquette étant susceptible de coopérer avec la partie centrale rigide (38) pour déplacer la paroi mobile (36).

14. Micropompe selon la revendication 1, caractérisée en ce que les moyens d'actionnement (450) comprennent un élément piézoélectrique (452) monté directement sur ladite paroi mobile (436).

15. Micropompe selon la revendication 8 ou 14, caractérisée en ce que la paroi mobile (36,436) comprend une partie centrale rigide (38,438),agencée de façon à entrer en contact avec ladite troisième plaquette (8) pour constituer un élément de butée limitant le mouvement d'aspiration du fluide de la paroi mobile (36,436).

16. Micropompe selon l'une des revendications précédentes, caractérisée en ce que la paroi mobile (436) présente dans la position de repos une forme bombée vers l'extérieur de la chambre de pompage (14).

17. Micropompe selon la revendication 16, caractérisée en ce que la hauteur de la forme bombée correspond sensiblement au déplacement de pompage effectué par la paroi mobile (436).

18. Micrompompe selon la revendication 16, caractérisée en ce que la forme bombée de la paroi mobile (436) est produite par la sous-pression existant dans une chambre (460) située sur la face arrière de la paroi mobile (436) et/ou par la présence d'au moins une couche d'un matériau appliqué sur la surface de la paroi mobile (436) induisant une pré-contrainte de déformation.

19. Micropompe selon l'une des revendications précédentes, caractérisée en ce que la face de la paroi mobile (36,436) qui est dirigée vers l'intérieur de la chambre de pompage (14) est destinée à venir buter contre la plaquette (2) située en regard pour limiter le mouvement d'expulsion du fluide.

20. Micropompe selon la revendication 1, caractérisée en ce que les faces de la paroi mobile (36) comprennent des zones (42, 44) constituées dans un matériau destiné à éviter un collage de ladite face contre la plaquette (2, 8) située en regard.

21. Micropompe selon l'une des revendications précédentes, caractérisée en ce que les organes de contrôle d'entrée et/ou de sortie du fluide sont constitués par au moins un clapet (12) comportant deux membranes (18) usinées dans la seconde plaquette (6) de façon à constituer une forme en V dans la position fermée du clapet, ces membranes (18) étant susceptibles de se séparer pour former une ouverture médiane (20) dans la position ouverte du clapet.

22. Micrompompe selon l'une des revendications précédentes, caractérisée en ce que les organes de contrôle d'entrée et/ou de sortie du fluide sont constitués par au moins une valve (412) en un matériau usiné par microusinage de surface.

23. Micropompe selon la revendication 8 ou 13, caractérisée en ce que l'organe de contrôle de sortie du fluide comprend une membrane de clapet (228) présentant une nervure annulaire (226) sollicitée contre une plaquette (2) située en regard et entourant la sortie (5) de la micropompe, cette dernière comportant au moins une chambre (229) délimitée par la face de la membrane de clapet (228) opposée à la nervure annulaire (226) et la troisième plaquette (208) ou la plaquette de base, cette chambre (229) étant destinée à former une cavité active ou de contrôle de la micropompe.

24. Micropompe selon l'une des revendications 1 à 23, caractérisée par le fait qu'elle est agencée pour l'administration de médicaments et susceptible d'être implantée dans le corps d'un patient.

## Patentansprüche

1. Mikropumpe mit zumindest einem Basisplättchen (2) und einem zweiten, so auf das Basisplättchen aufgeklebten Plättchen (6), dass eine Pumpenkammer (14) definiert wird; mit Regelorganen für den Eintritt (12) und Austritt (16) in direkter Verbindung mit der Pumpenkammer, wobei die benannten Regelorgane auf oder in zumindest einem der beiden Plättchen sitzen und zumindest eines dieser Plättchen durch photolithographische Mikrobearbeitungstechniken aus einem undurchlässigen Material herausgearbeitet wurde; und mit einem das Volumen der Pumpenkammer (14) und die Volumina der Verbindungsräume (22, 24) zu den benannten Regelorganen umfassenden inneren Volumen (Vi), wobei die Pumpenkammer (14) eine in eines der Plättchen eingearbeitete bewegliche Wand (36) hat und Betätigungsorgane (50) vorgesehen sind, um die benannte bewegliche Wand (36) zu verschieben und eine periodische Verkleinerung und Vergrösserung des inneren Volumens hervorzurufen; dadurch gekennzeichnet, dass sich das Regelorgan für den Eintritt (12) nach der Aussenseite des Plättchens hin öffnet, auf oder in dem es sitzt, während sich das Regelorgan für den Austritt (16) nach der Innenseite des Plättchens hin öffnet, auf oder in dem es sitzt, so dass die Verkleinerung des inneren Volumens ein in dem benannten inneren Volumen enthaltenes Gas auf einen Druck komprimiert, der dafiir ausreicht, die Öffnung des Austrittsregelorgans hervorzurufen und auf diese Weise eine selbstansaugende Mikropumpe zu bewirken.

2. Mikropumpe gemäss Anspruch 1, dadurch gekennzeichnet, dass die Pumpenkammer (14) und die Verbindungsräume (22, 24) so gestaltet sind, dass die auf Grund der Vergrösserung des Pumpenkammervolumens erfolgende Vergrösserung des inneren Volumens der Mikropumpe derart ist, dass das nach dem Schliessen des Fluidaustrittsregelorgans (16) in der Mikropumpe verbleibende Gas auf einen Druck entspannt wird, der niedrig genug ist, um die Öffnung des Fluideintrittsregelorgans (12) hervorzurufen.

3. Mikropumpe gemäss Anspruch 1 oder 2, dadurch gekennzeichnet, dass die Volumenverkleinerung (ΔVp) der Pumpenkammer zwischen 30 und 100 % und bevorzugt mehr als 50 % des benannten inneren Volumens (Vi) ausmacht, wobei die Volumina (Ve und Vs) der benannten Verbindungsräume zwischen der Pumpenkammer (14) und den Regelorganen für den Eintritt (12) und Austritt (16) weniger als 30 % des inneren Volumens (Vi) und bevorzugt weniger als 15 % davon betragen.

4. Mikropumpe gemäss Anspruch 1, dadurch gekennzeichnet, dass die Pumpenkammer (14) auf der einen Seite einer Mittelfläche des Plättchens angeordnet ist, in oder auf dem sie herausgearbeitet worden ist, wobei jedes der Regelorgane für den Eintritt (12) und Austritt (16) in oder auf der Seite des Plättchens, aus dem es herausgearbeitet wurde, angeordnet ist, die sich näher an der Pumpenkammer befindet.

5. Mikropumpe gemäss einem der vorangehenden Ansprüche, dadurch gekennzeichnet, dass das oder die Organe für den Eintritt (12) anders als das oder die Organe für den Austritt (16) gebaut sind.

6. Mikropumpe gemäss einem der vorangehenden Ansprüche, dadurch gekennzeichnet, dass die bewegliche Wand (36) eine starre zentrale Partie (38) umfasst, die von einer elastischen Kante (40) geringerer Wandstärke umgeben ist, die zusammen mit der starren zentralen Partie (38) aus einem Stück herausgearbeitet wurde, wobei diese letztere über die Seite der beweglichen Wand hinausragt, die der Pumpenkammer (14) gegenübersteht, und dazu bestimmt ist, mit den benannten Betätigungsorganen (50) zusammenzuwirken.

7. Mikropumpe gemäss Anspruch 6, dadurch gekennzeichnet, dass die Weite der benannten starren zentralen Partie (38) zwischen 20 und 90 %, vorzugsweise zwischen 50 und 80 % der Weite der beweglichen Wand (36) schwankt.

8. Mikropumpe gemäss einem der vorangehenden Ansprüche, dadurch gekennzeichnet, dass sie zumindest ein drittes, auf das zweite Plättchen (6) aufgeklebtes Plättchen (8) aufweist und die Betätigungsorgane (50) ein beweglich auf das dritte Plättchen montiertes Antriebselement (52) umfassen, wobei ein Zwischenstück (64) zwischen der benannten starren zentralen Partie (38) und dem Antriebselement (52) angeordnet ist.

9. Mikropumpe gemäss Anspruch 8, dadurch gekennzeichnet, dass das Antriebselement (52) beweglich auf die Aussenseite des benannten dritten Plättchens (8) montiert ist, wobei das benannte Zwischenstück (64) durch eine Öffnung (69) durch dieses dritte Plättchen (8) hindurchgeht.

10. Mikropumpe gemäss Anspruch 9, dadurch gekennzeichnet, dass das Antriebselement ein piezoelektrisches Element (52) ist, das vermittels eines Abstandshalters (62) auf die Aussenseite des dritten Plättchens (8) montiert ist.

11. Mikropumpe gemäss einem der Ansprüche 8 bis 10, dadurch gekennzeichnet, dass das Zwischenstiick (64) einen mit dem Antriebselement (52) fest verbundenen flachen Kopf (66) und einen Schaft (68) aufweist, der durch das dritte Plättchen (8) hindurchgeht und mit seinem Ende auf die bewegliche Wand (36) wirkt.

12. Mikropumpe gemäss Anspruch 8, dadurch gekennzeichnet, dass das Zwischenstück (164) eine Unterseite aufweist, die dafür bestimmt ist, mit der benannten starren zentralen Partie (38) in Berührung zu kommen, und eine Oberfläche darbietet, die der der starren zentralen Partie (38) ähnelt.

13. Mikropumpe gemäss Anspruch 6, dadurch gekennzeichnet, dass sie ein drittes Plättchen (208) aufweist, dessen Dicke seine elastische Deformation durch die Betätigungsvorrichtung (250) gestattet, die auf die Aussenseite des dritten Plättchens (208) montiert ist, wobei die Innenseite dieses dritten Plättchens dafiir geeignet ist, mit der starren zentralen Partie (38) zusammenzuwirken, um die bewegliche Wand (36) zu verschieben.

14. Mikropumpe gemäss Anspruch 1, dadurch gekennzeichnet, dass die Betätigungsorgane (450) ein direkt auf die benannte bewegliche Wand (436) montiertes piezoelektrisches Element (452) umfassen.

15. Mikropumpe gemäss Anspruch 8 oder 14, dadurch gekennzeichnet, dass die bewegliche Wand (36, 436) eine starre zentrale Partie (38, 438) umfasst, die so ausgeführt ist, dass sie mit dem benannten dritten Plättchen (8) in Berührung kommen kann, um ein Anschlagelement zu bilden, dass die Fluidansaugbewegung der beweglichen Wand (36, 436) begrenzt.

16. Mikropumpe gemäss einem der vorangehenden Anspriiche, dadurch gekennzeichnet, dass die bewegliche Wand (436) in ihrer Ruhestellung eine in Richtung auf die Aussenseite der Pumpenkammer (14) hin bauchige Gestalt darbietet.

17. Mikropumpe gemäss Anspruch 16, dadurch gekennzeichnet, dass die Höhe der Ausbauchung im wesentlichen der durch die bewegliche Wand (436) ausgeführten Pumpbewegung entspricht.

18. Mikropumpe gemäss Anspruch 16, dadurch gekennzeichnet, dass die bauchige Form der beweglichen Wand (436) durch den in einer auf der Rückseite der beweglichen Wand (436) befindlichen Kammer (460) existierenden Unterdruck und/oder durch die Gegenwart von zumindest einer auf die Oberfläche der beweglichen Wand (436) aufgebrachten Materialschicht hervorgerufen wird, die eine verformende Vorspannung erzeugt.

19. Mikropumpe gemäss einem der vorangehenden Ansprüche, dadurch gekennzeichnet, dass die dem Inneren der Pumpenkammer (14) zugewandte Seite der beweglichen Wand (36, 436) dazu bestimmt ist, an dem gegenüber befindlichen Plättchen (2) anzustossen, um die Fluidausstossbewegung zu begrenzen.

20. Mikropumpe gemäss Anspruch 1, dadurch gekennzeichnet, dass die Seiten der beweglichen Wand (36) Zonen (42, 44) haben, die aus einem Material bestehen, das dazu dient, ein Zusammenkleben der benannten Seite mit dem gegenüberliegenden Plättchen (2, 8) zu vermeiden.

21. Mikropumpe gemäss einem der vorangehenden Ansprüche, dadurch gekennzeichnet, dass die Regelorgane für den Fluideintritt und/oder -austritt aus zumindest einer Klappe (12) mit zwei Membranen (18) bestehen, die so aus dem zweiten Plättchen (6) herausgearbeitet wurden, dass sie bei geschlossener Klappe eine V-Form bilden und sich zu trennen vermögen, um bei offener Klappe eine zentrale Öffnung (20) zu bilden.

22. Mikropumpe gemäss einem der vorangehenden Ansprüche, dadurch gekennzeichnet, dass die Regelorgane für den Fluideintritt und/oder -austritt aus zumindest einem Ventil (412) aus einem durch Oberflächenmikrobearbeitung verformten Material bestehen.

23. Mikropumpe gemäss Anspruch 8 oder 13, dadurch gekennzeichnet, dass das Regelorgan für den Fluidaustritt eine Klappenmembran (228) umfasst, die eine ringförmige Rippe (226) aufweist, die gegen ein gegenüber befindliches Plättchen (2) gedrückt wird und den Austritt (5) der Mikropumpe umgibt, wobei diese letztere zumindest eine Kammer (229) aufweist, die vom dritten Plättchen (208) oder vom Basisplättchen und von der Seite der Klappenmembran (228) begrenzt wird, die der Seite mit der Ringrippe (226) entgegengesetzt ist, wobei diese Kammer (229) dazu bestimmt ist, einen Aktiv- oder Steuerhohlraum der Mikropumpe zu bilden.

24. Mikropumpe gemäss einem der Ansprüche 1 bis 23, dadurch gekennzeichnet, dass sie dafür eingerichtet ist, Medikamente zu verabreichen, und sich dafür eignet, in den Körper eines Patienten eingepflanzt zu werden.

## Claims

1. A micropump including at least one base platelet (2) and a second platelet (6) adhered to the base platelet, in such a manner as to define a pumping chamber (14), inflow (12) and outflow (16) control members directly connected with the pumping chamber, said control members being situated on or in at least one of the two platelets, at least one of these platelets being machined by micromachining techniques of photolithography, out of a impervious material; an internal volume (Vi) including the volume of the pumping chamber (14) and the volumes of the connecting spaces (22, 24) with said control members, the pumping chamber (14) including a movable wall (36) machined in one of the platelets, actuator means (50) being provided to move said movable wall (36) to produce periodically a decrease and an increase of the internal volume, characterized in that the inflow control member (12) opens outwards of the platelet on which or in which it is located, whereas the outflow control member (16) opens inwards of the platelet on which or in which it is located, so that the decrease in the internal volume compresses a gas contained in said internal volume to a pressure sufficient for producing the opening of the outflow control member, so as to obtain a self-priming pump.

2. A micropump according to claim 1, characterized in that the pumping chamber (14) and the connection spaces (22, 24) are designed in such a manner that the increase of the internal volume of the micropump, due to the increase in volume of the pumping chamber, is such that the gas remaining in the micropump after the closure of the outflow control member (16) of the fluid is decompressed to a pressure sufficiently low to produce the opening of the fluid inflow control member (12).

3. A micropump according to claim 1 or 2, characterized in that the decrease in volume (ΔVp) of the pumping chamber is comprised between 30 and 100% of the internal volume (Vi), preferably more than 50%, the volumes (Ve and Vs) of said connection spaces of the pumping chamber (14) to the inflow (12) and outflow (16) control members being less than 30% of the internal volume (Vi), preferably less than 15%.

4. A micropump according to claim 1, characterized in that the pumping chamber (14) is arranged on one side of a medial surface of the platelet in which or on which it is micromachined, each one of the inflow (12) and outflow (16) members being arranged in or on that side of the platelet on which it is machined, which is the closest to the pumping chamber.

5. A micropump according to one of the preceding claims, characterized in that the inflow member or members (12) are of a different construction from that of the outflow member or members (16).

6. A micropump according to one of the preceding claims, characterized in that the movable wall (36) includes a central rigid part (38) surrounded by a resilient rim (40) of a smaller thickness, integral with the central rigid part (38), the latter protruding with respect to the face of the movable wall which is opposite to the pumping chamber (14) and being designed for cooperating with said actuator means (50).

7. A micropump according to claim 6, characterized in that the width of said central rigid part (38) amounts to between 20 and 90% of the width of the movable wall (36), preferably between 50 and 80%.

8. A micropump according to one of the preceding claims, characterized in that it exhibits at least a third platelet (8) adhered to the second platelet (6), the actuator means (50) include a motor member (52) mounted movably on the third platelet, an intermediate part (64) being positioned between said central rigid part (38) and the motor member (52).

9. A micropump according to claim 8, characterized in that the motor member (52) is mounted movably on the outer face of said third platelet (8), said intermediate part (64) extending through this third platelet (8) via an opening (69).

10. A micropump according to claim 9, characterized in that the motor member is a piezoelectric member (52) which is mounted via a spacing member (62) on the outer face of the third platelet (8).

11. A micropump according to one of claims 8 to 10, characterized in that the intermediate part (64) includes a flat head (66) integral with the motor member (52) and a rod (68) extending through the third platelet (8) and acting through its end on the movable wall (36).

12. A micropump according to claim 8, characterized in that the intermediate part (164) has a lower face designed for coming in contact with said central rigid part (38) exhibiting a surface similar to that of the central rigid part (38).

13. A micropump according to claim 6, characterized in that it includes a third platelet (208) having a thickness such that it can be deformed elastically by the actuator device (250) which is mounted on the outer face of the third platelet (208), the inner face of this third platelet being capable of cooperating with the central rigid part (38) to move the movable wall (36).

14. A micropump according to claim 1, characterized in that the actuator means (450) include a piezoelectric member (452) mounted directly on said movable part (436).

15. A micropump according to claim 8 or 14, characterized in that the movable wall (36, 436) includes a central rigid part (38, 438) arranged in such a manner as to come in contact with said third platelet (8) to form a stop member limiting the fluid suction motion of the movable wall (36, 436).

16. A micropump according to one of the preceding claims, characterized in that the movable wall (436) has, in the rest position, the shape of a dome directed outwards of the pumping chamber (14).

17. A micropump according to claim 16, characterized in that the height of the dome shape corresponds substantially to the pumping displacement of the movable wall (436).

18. A micropump according to claim 16, characterized in that the dome shape of the movable wall (436) is produced by the depression prevailing in a chamber (460) situated on the back face of the movable wall (436) and/or by the presence of at least one layer of a material applied on the surface of the movable wall (436) inducing a deformation stress.

19. A micropump according to one of the preceding claims, characterized in that the face of the movable wall (36, 436) directed towards the inside of the pumping chamber (14) is designed for abutting against the facing platelet (2) to limit the movement of expulsion of the fluid.

20. A micropump according to claim 1, characterized in that the faces of the movable wall (36) include areas (42,44) made of a material designed for avoiding an adhesion of said face against the facing platelet (2, 8).

21. A micropump according to one of the preceding claims, characterized in that the inflow and/or outflow control members are comprised of at least one valve (12) including two membranes (18) machined in the second platelet (6) in such a manner as to assume the shape of a V in the closed position of the valve, these membranes (18) being capable of separating to form a medial opening (20) in the open position of the valve.

22. A micropump according to one of the preceding claims, characterized in that the inflow and/or outflow control members are comprised of at least one valve (412) of a material formed by surface micromachining.

23. A micropump according to claim 8 or 13, characterized in that the outflow control member includes a valve membrane (228) exhibiting an annular rib (226) biased against a facing platelet (2) and surrounding the outlet (5) of the micropump, the latter including at least a chamber (229) defined by the face of the valve membrane (228) opposite the annular rib (226) and the third platelet (208) or base platelet, this chamber (229) being designed for forming an active or a control cavity of the micropump.

24. A micropump according to one of claims 1 to 23, characterized in that it is used for the administration of a medicament and is capable of being implanted in the body of a patient.
